# EUROPÄISCHE PATENTSCHRIFT

(11) **EP 1 706 059 B1**
(45) Veröffentlichungstag und Bekanntmachung des Hinweises auf die Patenterteilung: **12.10.2011**
(21) Anmeldenummer: 05700524.1
(22) Anmeldetag: 07.01.2005
(51) Int. Cl.: A61C 17/08, A61B 1/247

(54) **MEDIZINISCHER ABSAUGER**
MEDICAL SUCTION DEVICE
ASPIRATEUR MEDICAL

(30) Priorität: 09.01.2004 DE 102004001621; 05.11.2004 DE 102004054029
(43) Veröffentlichungstag der Anmeldung: 04.10.2006
(73) Patentinhaber: Clasen, Stephan, 48149 Münster (DE); Kayser, Martin, 50968 Köln (DE)
(72) Erfinder: Clasen, Stephan, 48149 Münster (DE); Kayser, Martin, 50968 Köln (DE)
(74) Vertreter: Kayser, Martin
(86) Internationale Anmeldenummer: PCT/DE2005/000011
(87) Internationale Veröffentlichungsnummer: WO 2005/065573

(56) Entgegenhaltungen:
- EP-A- 0 003 470
- WO-A-2004/034892
- DE-A1- 10 065 705
- FR-A- 2 595 939
- US-A- 5 490 780
- US-A- 5 743 736

## Beschreibung

Die vorliegende Erfindung betrifft einen zahnmedizinischen Absauger zum Absaugen von Flüssigkeiten und Partikeln aus einem Mundraum eines Patienten während einer Behandlung, mit einem hohlen Grundkörper mit einer Längsachse X-X, der eine Außenfläche, eine Innenfläche und eine Ansaugöffnung aufweist.

Bei medizinischen und zahnmedizinischen Behandlungen ist es oftmals notwendig, anfallende Flüssigkeiten oder gelöste Partikel vom Behandlungsbereich abzuführen. Beispielsweise ist es bei einer zahnmedizinischen Behandlung notwendig, Speichel, Spraywasser und Blut während der Behandlung abzusaugen. Auch kann Wasser, beispielsweise zum Reinigen oder nach Anwenden einer Multifunktionsspritze anfallen, das abgesaugt werden muss. Hierzu werden üblicherweise Absauger verwendet, die in der Regel aus einem röhrenförmigen Körper aus Kunststoff gebildet sind, an dessen Ende ein Schlauch befestigt ist, der wiederum mit einer Pumpe verbunden ist. Durch den Schlauch bzw. den Absauger werden dann die störenden Flüssigkeiten und Festkörper abgesaugt.

Auch in anderen medizinischen Bereichen, insbesondere bei chirurgischen Behandlungen ist es häufig notwendig, Blut, Wasser und/oder Knochenpartikel und ähnliches während der Operation abzusaugen.

Üblicherweise wird ein Absauger nicht vom behandelnden Arzt bzw. behandelnden Chirurgen selbst, sondern von einer oder einem Assistenten geführt und gehalten. Dies ist deshalb notwendig, weil der behandelnde Arzt meist beide Hände für seine Behandlung benötigt. Ein Zahnarzt führt z.B. oftmals mit der einen Hand ein Bohrwerkzeug und mit der anderen Hand einen Spiegel, über den er den zu behandelnden Bereich einsehen kann. Das gleiche gilt für einen Chirurgen, der während einer Operation ebenfalls oftmals zwei Geräte führt, aber dennoch einen Spiegel benötigt.

Nachteilig bei der beschrieben Vorgehensweise ist, dass zwei Personen, nämlich der behandelnde Arzt und die assistierende Person sehr eng beieinander an der zu behandelnden Person um den zu behandelnden Bereich stehen oder sitzen müssen. Dies kann gerade dann, wenn es sich um verhältnismäßig schwierige oder feinmotorisch anspruchsvolle Eingriffe handelt, für den behandelnden Arzt als störend empfunden werden.

Ein weiterer Nachteil ergibt sich daraus, dass der behandelnde Arzt, dann wenn er beispielsweise ein medizinisches Werkzeug, einen Absauger und einen Spiegel gebrauchen muss, auf eine assistierende Person angewiesen ist. Dies ist gerade bei Zahnärzten ein besonderer Nachteil, weil die zahnärztlichen Behandlungen oftmals problemlos ohne eine assistierende Person durchgeführt werden könnten, dies jedoch daran scheitert, dass Flüssigkeiten oder Partikel abgesaugt werden müssen.

Schließlich ist es oftmals störend, dass ein gattungsgemäßer Absauger oftmals dem Bohrer im Weg ist, also das Arbeiten mit Bohrer und Absauger gleichzeitig schwierig ist.

Die Druckschriften FR 2 595 939, US 5490 780 und WO 2004/034892 zeigen jeweils Absauger, bei denen vor der Ansaugöffnung des Absaugers ein Element mit einem Spiegel angeformt ist. Der Spiegel hilft zwar, die Sicht des Zahnarztes zu verbessern, allerdings ist der Raumbedarf störend und die Ansaugkraft des Absaugers ist reduziert.

Die Aufgabe der vorliegenden Erfindung besteht darin, einen gattungsgemäßen Absauger derart zu verbessern, dass er zum einen vielseitig einsetzbar ist, zum anderen dem Benutzer seine vorzunehmende Behandlung erleichtert. Eine weitere Aufgabe besteht darin, es dem Benutzer zu ermöglichen, bestimmte Arbeiten oder Behandlungen auch ohne eine assistierende Person durchführen zu können. Der Absauger soll dabei kostengünstig herstellbar und einfach einzusetzen sein. Weiterhin soll er leicht und möglichst ohne Nachteile für den Patienten benutzbar sein.

Erfindungsgemäß wird die Aufgabe durch einen zahnmedizinischen Absauger mit den Merkmalen des unabhängigen Patentanspruchss 1 gelöst.

Besonders vorteilhaft ist, wenn der Verlauf der Längsachse X-X eines solchen zahnmedizinischen Absaugers zum Absaugen von Flüssigkeiten und Partikeln aus einem Mundraum eines Patienten während einer Behandlung, der einen hohlen Grundkörper mit einer Längsachse, der eine Außenfläche, eine Innenfläche und eine Absaugöffnung aufweist, wobei der Grundkörper in Längsrichtung derart verformbar ausgeführt ist, in alle Richtungen veränderbar ist.

Die erfindungsgemäße spiegelnde Beschichtung ermöglicht es dem Benutzer, den medizinischen Absauger sowohl als Absauger zum Abführen von Flüssigkeiten und Partikeln, als auch gleichzeitig als Spiegel zu benutzen. Dies ist beispielsweise für einen Zahnarzt sehr hilfreich, da dieser beim Behandeln eines Zahns mit einem Bohrwerkzeug in der Regel gleichzeitig bohren und absaugen muss. Weiterhin muss er dann, wenn er die zu behandelnde Stelle nicht einsehen kann, einen Spiegel benutzen. Mit Hilfe des erfindungsgemäßen Absaugers ist es ihm nun möglich, die Behandlung ohne eine assistierende Person durchzuführen. Der Absauger wird also gleichzeitig als Spiegel verwendet.

Um die gewünschte Funktion erfüllen zu können, ist es nicht notwendig, den gesamten Absauger mit einer spiegelnden Beschichtung zu versehen, vielmehr reicht es aus, wenn diese im Bereich der Ansaugöffnung vorgesehen ist. Es kann sowohl die Innenfläche als auch die Außenfläche beschichtet sein.

Handelsübliche Absauger für zahnmedizinische Zwecke weisen in der Regel eine Ansaugöffnung auf, die sich schräg zur Hauptlängserstreckung des rohrförmigen Grundkörpers erstreckt. Das die Ansaugöffnung aufweisende Ende des rohrförmigen Grundkörpers verläuft also nicht rechtwinklig zur Längsachse, sondern ist schräg zu ihr ausgeführt. Dadurch ergibt sich eine schräg zulaufende Form des Absaugers, wodurch dieser leichter beispielsweise zwischen Wange und Zähne einführbar ist. In einem solchen Anwendungsfall ist dann die Ansaugöffnung dem Zahn zugewandt, während ein der Ansaugöffnung gegenüberliegender längerer Bereich des Grundkörpers an der Wange anliegt.

In einer besonders vorteilhaften Ausführungsform der Erfindung ist insbesondere die Innenfläche des Grundkörpers in dem Bereich spiegelnd beschichtet, der von außen einsehbar ist. Das bedeutet, dass der Absauger genau so, wie es der behandelnde Zahnarzt gewohnt ist, an den Zahn herangeführt wird, nämlich mit der Ansaugöffnung in Richtung des Zahns, und der Zahnarzt dann durch die Ansaugöffnung über die spiegelnde Innenfläche auf den Zahn bzw. behandelnden Bereich blicken kann. Der Absauger wird also durch den Spiegel nicht weiter verlängert. Dies hat den Vorteil, dass die Absaugleistung nicht negativ beeinflusst wird, da sich der Abstand zum abzusaugenden Bereich nicht vergrößert. Außerdem hat sich gezeigt, dass sich aus dem gleichen Grund die durch den Luftzug zwangsläufig entstehenden Geräusche nicht zunehmen. Dies ist bei einem der Ansaugöffnung vorgelagerten Spiegel der Fall, da dieser ungünstige Luftwirbel erzeugen kann.

Unabhängig davon kann es vorteilhaft sein; wenn anstelle oder zusätzlich zur Innenbeschichtung die Außenfläche des Grundkörpers spiegelnd beschichtet ist.

In einer weiteren Ausführungsform der Erfindung ist der Absauger in seinem die Ansaugöffnung aufweisenden Endbereich aufgeweitet ausgeführt, um dadurch insbesondere die innen liegende Spiegelfläche zu erhöhen. Dies ist dann sinnvoll, wenn die durch die Ansaugöffnung sichtbare Innenfläche des Absaugers als Spiegelfläche genutzt wird. Auch ermöglicht die Aufweitung ein besseres Abhalten von beispielsweise Weichgewebe und ähnlichem.

Je nach gewünschter Funktion kann es sinnvoll sein, wenn die spiegelnde Beschichtung eine vergrößernde oder verkleinernde Wirkung hat. Diese kann zum einen dadurch erreicht werden, dass der Absauger selbst eine konvexe oder konkave Form aufweist, die dann mit einem entsprechenden reflektierenden bzw. spiegelnden Material beschichtet wird, oder dadurch, dass auf den Grundkörper beispielsweise ein Hohlspiegel oder ein konvexer Spiegel aufgebracht wird. Dieser kann aufgeklebt sein, er kann aber auch in das Material, das den Grundkörper ausbildet, eingeformt sein. Wesentlich ist, dass dann, wenn ein zusätzlicher Spiegel verwendet wird, dieser derart mit dem Grundkörper verbunden wird, dass eine Desinfektion des Absaugers problemlos möglich ist. Es bieten sich hierzu beispielsweise die Verfahren an, die verwendet werden, um bei einem üblichen Zahnarztspiegel den Spiegel mit dem haltenden Grundkörper zu verbinden.

Besonders vorteilhaft ist die Verwendung eines Ansteckelementes, das die spiegelnde Oberfläche aufweist. Dieses ist im Bereich der Ansaugöffnung mit dem Absauger lösbar verbunden. Beispielsweise kann der Absauger eine Nut aufweisen, in das Ansteckelement einsetzbar ist. Dadurch wird erreicht, dass das Ansteckelement beliebig austauschbar ist und an gewünschte Situationen angepasst werden kann. Das Ansteckelement kann beliebige Formen aufweisen, die spiegelnde Oberfläche kann konkav, konvex oder andersartig ausgeführt sein.

Die erfindungsgemäßen Absauger können aus jedem geeigneten Material hergestellt sein, es bietet sich jedoch aus Kostengründen eine Fertigung aus einem Kunststoffmaterial an.

Erfindungsgemäß ist der Grundkörper derart ausgeführt, dass er durch plastische Verformung an die gewünschten Gegebenheiten anpassbar ist. Dies kann beispielsweise dadurch erreicht werden, dass der Grundkörper aus einem verhältnismäßig weichem, plastisch verformbaren Kunststoff hergestellt ist, der dann vom behandelnden Arzt vor dem bestimmungsgemäßen Gebrauch durch einfaches Verbiegen in die gewünschte Form gebracht wird. Alternativ bietet sich auch die Verwendung eines oder mehrerer Kugelgelenke an. Wesentlich ist, dass der Verlauf der Längsachse des Absaugers in alle Richtungen veränderbar ist. Bei einer besonders vorteilhaften Ausführung ist ein solches Gelenk nahe an dem spiegelnden Bereich angeordnet oder der spiegelnder Bereich selbst ist um die Längsachse drehbar und oder zu ihr schwenkbar ausgeführt.

Anhand der nachfolgenden Figuren wird die Erfindung näher erläutert.

Es zeigen:
- Fig. 1:: eine Prinzipdarstellung eines erfindungsgemäßen Absaugers in Seitenansicht,
- Fig. 2:: eine vergrößerte Darstellung desjenigen Endbereichs des Absaugers, der eine Ansaugöffnung aufweist.

Wie sich aus den beiden Figuren ergibt, weist ein medizinischer Absauger 10 einen hohlen, beispielsweise rohrförmigen Grundkörper 12 mit einer Innenfläche 14 und einer Außenfläche 16 auf. Weiterhin weist der Grundkörper 12 eine Längsachse X-X auf, die gerade, oder wie im Ausführungsbeispiel gezeigt, mit einem Knick verlaufen kann: Der Knick hat den Vorteil, dass der Absauger 10 leichter an die zu behandelnde Stelle gebracht werden kann.

Der Grundkörper 12 weist eine Anschlussöffnung 18 und eine Ansaugöffnung 20 auf. Die Anschlussöffnung 18 dient zum Anschluss an einen hier nicht gezeigten Schlauch, durch die Ansaugöffnung 20 werden die abzusaugenden Flüssigkeiten oder Partikel eingesogen.

Im vorliegenden Ausführungsbeispiel ist weiterhin ein Rand 22 gezeigt, der sich um Bereiche der Ansaugöffnung 20 herum erstreckt. Dieser Rand 22 ist für einen Absauger 10, der im zahnmedizinischen Bereich eingesetzt wird, die Wange oder Lippe des Patienten besser von dem zu behandelnden Bereich abhalten zu können. Weiterhin wird dieser diesen Rand 22, dass keine scharfkantigen Kanten im Bereich der Ansaugöffnung vorhanden sind.

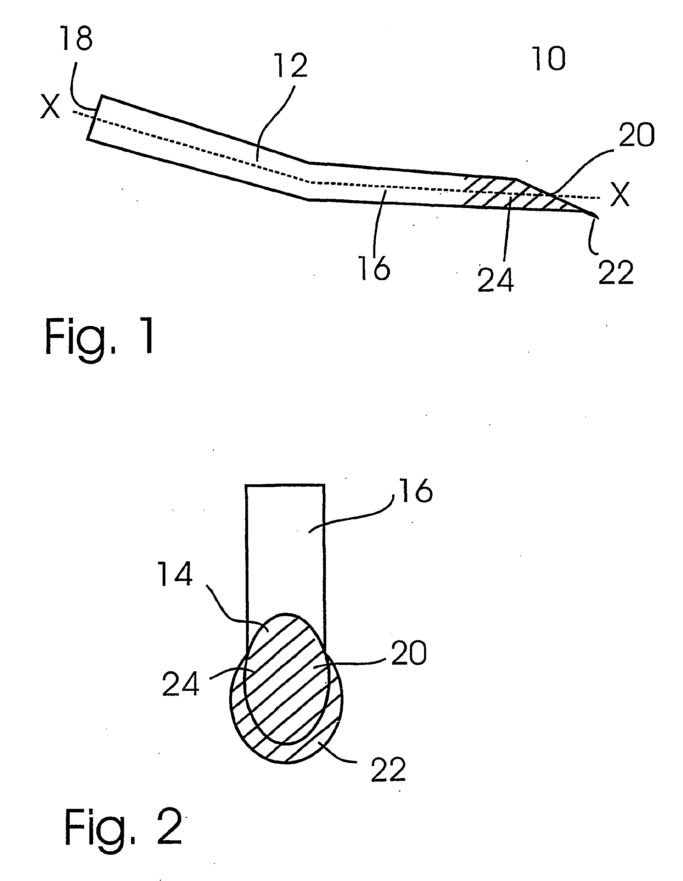

Erfindungsgemäß weisen Bereiche des Absaugers 10 eine spiegelnde Oberfläche 24 auf. Diese ist durch eine Schraffur gekennzeichnet. Es hat sich als vorteilhaft erwiesen, wenn beispielsweise, wie im gezeigten Ausführungsbeispiel, die Innenfläche 14 des Absaugers 10 in dem Bereich verspiegelt ausgeführt ist, der durch die Ansaugöffnung 20 einsehbar ist. Dies ist genau der Bereich, der bei der Behandlung beispielsweise dem zu behandelnden Zahn gegenüberliegt, da sie ohnehin für eine effektive Reinigung nahe an den Bereich herangebracht werden muss. Weiterhin ist durch den ständigen Luftzug, der sich durch das Ansaugen an der Innenfläche 14 des Grundkörpers 12 ergibt, gewährleistet, dass die spiegelnde Oberfläche 24 nicht beschlagen kann. Unabhängig davon ist es jedoch möglich, wie in Figur 1 dargestellt, auch Bereiche der Außenfläche 16 mit einer spiegelnden Oberfläche 24 zu versehen. Der Rand 22 kann vorzugsweise auch breiter als gezeigt ausgeführt sein, um die spiegelnde Oberfläche zu vergrößern.

Auch kann es sinnvoll sein, wenn alle sichtbaren Flächen des Grundkörpers 12 eine spiegelnde Oberfläche 24 aufweisen oder der Grundkörper 12 aus einem spiegelnden Material hergestellt ist.

Vorteilhafterweise kann der Absauger 10 im Bereich seiner Ansaugöffnung 20 eine Lichtquelle aufweisen, die den Bereich beleuchtet, der über die spiegelnde Oberfläche 24 sichtbar sein soll. Dies kann beispielsweise durch einen Lichtleiter gewährleistet sein, der sich entlang des Grundkörpers 12 erstreckt. Die Lichtquelle kann direkt im Bereich der Anschlussöffnung 18 oder sogar extern angeordnet sein kann, wenn sie in den Lichtleiter einstrahlt.

Der Grundkörper 12 kann aus allen geeigneten Materialien gefertigt sein, es bietet sich beispielsweise ein harter Kunststoff an, da dieser leicht zu desinfizieren ist. Der Grundkörper kann dann, um die erfindungsgemäße Verformbarkeit zu gewährleisten, lediglich bereichsweise aus weicherem Material bestehen oder Bereiche ähnlich einem Falgenbalg aufweisen. Ein Falgenbalg hat den Vorteil, dass der Absauger 10 auch verkürzt oder verlängert werden kann.

Der erfindungsgemäße Absauger 10 ermöglicht es der behandelnden Person, den Patienten von hinten (von 12 Uhr) zu behandeln. Dies entlastet den Rücken der behandelnden Person deutlich, da er sich von oben über den Patienten beugen kann. Da der Einsatz von Absaugern gerade im zahnmedizinischen Bereich sehr häufig ist, kann die Erfindung einen wertvollen Beitrag zur verbesserten Ergonomie am Arbeitsplatz leisten.

Die Erfindung ist nicht auf die beschriebenen Ausführungsbeispiele beschränkt, sondern sie erstreckt sich auf alle gleich wirkenden Ausführungsformen. Die beschriebene Ausführungsvariante ist nur beispielhaft und nicht einschränkend zu verstehen.

## Patentansprüche

1. Zahnmedizinischer Absauger (10) zum Absaugen von Flüssigkeiten und Partikeln aus einem Mundraum eines Patienten während einer Behandlung, mit einem hohlen Grundkörper (12) mit einer Längsachse X-X, der eine Außenfläche (16), eine Innenfläche (14) und eine Ansaugöffnung (20) aufweist, **dadurch gekennzeichnet, dass** die Innenfläche (14) eine durch die Ansaugöffnung (20) einsehbare verspiegelte Oberfläche (24) aufweist.

2. Zahnmedizinischer Absauger (10) nach Anspruch 1, **dadurch gekennzeichnet, dass** die Ansaugöffnung (20) derart schräg zur Längsachse X-X ausgeführt ist, dass die Größe der Ansaugöffnung (20) eine quer zur Längsachse verlaufende Querschnittsfläche des Grundkörpers (12) übersteigt.

3. Zahnmedizinischer Absauger (10) nach Anspruch 1 oder Anspruch 2, **dadurch gekennzeichnet, dass** auch die Außenfläche (16) des Grundkörpers (12) eine spiegelnde Oberfläche (24) aufweist.

4. Zabnmedizinischer Absauger (10) nach einem der Ansprüche 1 bis 3, **dadurch gekennzeichnet, dass** der Grundkörper (12) in Längsrichtung derart verformbar ausgeführt ist, dass der Verlauf der Längsachse X-X veränderbar ist.

5. Zahnmedizinischer Absauger (10) nach Anspruch 4, **dadurch gekennzeichnet, dass** der Grundkörper (12) aus einem biegsamen plastischem Material gefertigt ist.

6. Zahnmedizinischer Absauger (10) nach einem der Ansprüche 1 bis 5, **dadurch gekennzeichnet, dass** im Bereich der Ansaugöffnung (20) eine Lichtquelle derart angeordnet ist, dass der zu spiegelnde Bereich beleuchtbar ist.

7. Zahnmedizinischer Absauger (10) nach einem der Ansprüche 1 bis 6, **dadurch gekennzeichnet, dass** der Absauger (10) derart ausgeführt ist, dass er für chirurgische Zwecke einsetzbar ist.

## Claims

1. Dentistry suction device (10) for sucking liquids and particles out of an oral cavity of a patient during a treatment, comprising a hollow basic member (12) with a longitudinal axis X-X which has an external surface (16), an internal surface (14) and a suction port (20), **characterised in that** the internal surface (14) comprises a mirrored surface (24) that is visible through the suction port (20).

2. Dentistry suction device (10) according to claim 1, **characterised in that** the suction port (20) Is configured with such an inclination to the longitudinal axis X-X that the size of the suction port (20) exceeds a cross-sectional surface area of the basic member (12) extending transversely to the longitudinal axis.

3. Dentistry suction device (10) according to claim 1 or claim 2, **characterised in that** the external surface (16) of the basic member (12) also has a reflecting surface (24).

4. Dentistry suction device (10) according to any one of the claims 1 to 3, **characterised In that** the basic member (12) is configured to be deformable In the longitudinal direction In such a way that the course of the longitudinal axis X-X can be changed.

5. Dentistry suction device (10) according to claim 4, **characterised in that** the basic member (12) is made from a flexible plastic material.

6. Dentistry suction device (10) according to any one of the claims 1 to 5, **characterised in that** a light source is disposed in the area of the suction port (20) In such a way that the area to be reflected can be Illuminated.

7. Dentistry suction device (10) according to any one of the claims 1 to 6, **characterised in that** the suction device (10) is configured so that It can be used for surgical purposes.

## Revendications

1. Aspirateur de médicine dentaire (10) destiné à aspirer des liquides et particules d'un espace buccal d'un patient durant un traitement, comprenant un corps de base creux (12) ayant un axe longitudinal X-X qui présente une face extérieure (16), une face intérieure (14) et une ouverture d'aspiration (20), **caractérisé par le fait que** ladite face intérieure (14) présente une surface spéculaire (24) visible depuis ladite ouverture d'aspiration (20).

2. Aspirateur de médecine dentaire (10) selon la revendication 1, **caractérisé par le fait que** ladite ouverture d'aspiration (20) est réalisée de manière à être oblique par rapport à l'axe longitudinal X-X d'une telle façon que la dimension de ladite ouverture d'aspiration (20) dépasse une aire de la section du corps de base (12), s'étendant transversalement à l'axe longitudinal.

3. Aspirateur de médecine dentaire (10) selon la revendication 1 ou la revendication 2, **caractérisé par le fait que** la face extérieure (16) du corps de base (12) présente, elle aussi, une surface spéculaire (24).

4. Aspirateur de médecine dentaire (10) selon l'une quelconque des revendications 1 à 3, **caractérisé par le fait que** ledit corps de base (12) est réalisé de manière à être déformable dans la direction longitudinale d'une telle façon que le tracé de l'axe longitudinal X-X soit modifiable.

5. Aspirateur de médecine dentaire (10) selon la revendication 4, **caractérisé par le fait que** ledit corps de base (12) est réalisé dans une matière plastique flexible.

6. Aspirateur de médecine dentaire (10) selon l'une quelconque des revendications 1 à 5, **caractérisé par le fait qu'**une source de lumière est disposée au niveau de ladite ouverture d'aspiration (20) de manière à ce que la zone à réfléchir puisse être éclairée.

7. Aspirateur de médecine dentaire (10) selon l'une quelconque des revendications 1 à 6, **caractérisé par le fait que** ledit aspirateur (10) est réalisé de manière à ce qu'il soit utilisable à des fins chirurgicales.
